# EUROPEAN PATENT SPECIFICATION

(11) **EP 0 891 967 B1**
(45) Date of publication and mention of the grant of the patent: **27.09.2006**
(21) Application number: 97305416.6
(22) Date of filing: 18.07.1997
(51) Int. Cl.: C07C 253/34

(54) **Improved acrylonitrile recovery process**
Verfahren zur Wiedergewinnung von Acrylonitril
Procédé de récupération d'acrylonitrile

(43) Date of publication of application: 20.01.1999
(73) Proprietor: Innovene USA LLC, Chicago, IL 60601 (US)
(72) Inventor: Godbole, Sanjay Parushottam, Solon, Ohio 44139 (US); Wachtendorf, Paul Trigg, Wapakoneta, Ohio 45895 (US); Rinker, Jeffrey Earle, Elida, Ohio 45807 (US)
(74) Representative: Preece, Michael

(56) References cited:
- EP-A- 0 024 788
- GB-A- 1 271 200
- US-A- 3 399 120
- US-A- 3 885 928

## Description

The present invention is directed to an improved process for the manufacture of acrylonitrile or methacrylonitrile. In particular, the present invention is directed to the improvement in the recovery procedures utilized during the manufacture of acrylonitrile and methacrylonitrile.

Recovery of acrylonitrile/methacrylonitrile produced by the ammoxidation of propylene or isobutylene on a commercial scale has been accomplished by quenching the reactor effluent with water followed by passing the gaseous stream containing acrylonitrile or methacrylonitrile resulting from the quench to an absorber where water and the gases are contacted in counter-current flow to remove substantially all the acrylonitrile or methacrylonitrile, the aqueous stream containing substantially all the acrylonitrile or methacrylonitrile is then passed through a series of distillation columns and associated decanters for separation and purification of product acrylonitrile or methacrylonitrile.

Typical recovery and purification systems that are used during the manufacture of acrylonitrile or methacrylonitrile are disclosed in U.S. Patent Nos. 4,234,510 and 3,885,928, assigned to the assignee of the present invention and herein incorporated by reference.

US 3399120 relates to the purification of olefinically unsaturated nitriles by water extractive distillation. It teaches the use of an improvement involving a particular configuration of two distillation columns. According to column 7 at lines 61 to 64, the pressure in the entire system including both columns is usually at or near atmospheric pressures ie from atmospheric to 10 psig.

It is the primary object of the present invention to provide an improved process for the manufacture of acrylonitrile or methacrylonitrile.

It is another object of the present invention to provide an improved recovery and purification procedure for utilization during the manufacture of acrylonitrile or methacrylonitrile.

It is a further object of the present invention to provide an improved process for the manufacture of acrylonitrile or methacrylonitrile which reduces waste gas and improves the product throughput, recovery efficiency and product quality by reducing the amount of organic impurities in the resultant final product.

Additional objects, advantages and novel features of the invention will be set forth in part in the description which follows, and in part, will become apparent to those skilled in the art upon examination of the following or may be learned by the practice of the invention. The objects and advantages of the invention may be realized and obtained by means of the instrumentalities and combinations particularly pointed out in the appended claims.

To achieve the foregoing and other objects and in accordance with the purpose of the present invention as embodied and broadly described herein, the process of the present invention comprises transporting the reactor effluent obtained during the ammoxidation of propylene or isobutylene to a quench column wherein the hot effluent gases are cooled by contact with an aqueous spray, the cooled reactor effluent is then passed overhead to an absorber column wherein the acrylonitrile or methacrylonitrile is absorbed in water, the aqueous solution containing the acrylonitrile or methacrylonitrile is then passed through a recovery distillation column and stripper distillation column to recover the acrylonitrile or methacrylonitrile product wherein the improvement comprises increasing the recovery distillation column top pressure by mechanical means by about 0.7 to 34.5 kPa (0.1 to 5 psi) to improve the hydraulic capacity of the recovery and stripper columns. For example, typically the recovery column top pressure in the recovery and purification section of an acrylonitrile plant is designed to run at between about 108 to less than 136 kPa (1 to less than 5 psig). The present invention is directed to mechanically, for example by the addition of a pressure valve, increasing the top pressure of the recovery column above the designed pressure by between 0.7 to 34.5 kPa (0.1 to 5 psi), preferably 1.7-3.5 to 34.5 kPa (0.25-0.5 to 5.0), especially preferred being 7 to 34.5 kPa (1.0 to 5.0 psi).

In a preferred embodiment of the present invention, the recovery column top pressure is maintained at between 136 to 170 kPa (5 to 10 psig), preferably greater than 136 to 170 kPa (5 to 10 psig), especially preferred being 139 to 153 kPa (5.5 to 7.5 psig).

In a preferred embodiment of the present invention, the process is performed with the reactor effluent obtained from the ammoxidation of propylene, ammonia and oxygen to produce acrylonitrile.

In a still preferred embodiment of the present invention, the reactor effluent is obtained by the reaction of propylene, ammonia and air in a fluid bed reactor while in contact with a fluid bed catalyst.

Conventional fluid bed ammoxidation catalyst may be utilized in the practice of the invention. For example, fluid bed catalyst as described in U.S. Patent Nos. 3,642,930 and 5,093,299, may be utilized in the practice of the present invention.

In conventional recovery and purification procedures for the recovery of acrylonitrile or methacrylonitrile, it has been found that jet flooding in the recovery and stripper columns typically sets the maximum amount of lean water which can be circulated through the absorber, recovery and stripper columns. The present invention allows the operation of the recovery and stripper columns at increased pressure, thus increasing the throughput at which jet flooding occurs, thereby allowing for an increase in the rate of reactor operation and lower absorber off-gas losses. This is accomplished by increasing the flow of the lean water from the recovery and stripper columns to the absorber with the surprising discovery that the total recovery procedure does not lose any operating efficiency . The practice of the invention allows for higher reaction production rates while maintaining the minimum requirements as to the lean water to product ratio (at least 11:1). The practice of the present invention is very beneficial when utilized in combination with a recovery and purification procedure which operates at a lean water to product ratio of 12:1 to 11:1 in the absorber column.

The present invention will now be described in detail. The reactor effluent obtained by the ammoxidation of propylene or isobutylene, ammonia and oxygen containing gas in a fluid bed reactor while in contact with a fluid bed ammoxidation catalyst is transported to a quench column wherein the hot effluent gases are cooled by contact with water spray. Typically, any excess ammonia contained in the effluent is neutralized by contact with sulfuric acid in the quench to remove the ammonia as ammonium sulfate. The cooled effluent gas containing the desired product (acrylonitrile or methacrylonitrile and HCN) is then passed into the bottom of an absorber column wherein the products are absorbed in water which enters the column from the top. The non-absorbed gases pass from the absorber through a pipe located at the top of the absorber. The aqueous stream containing the desired product is then passed from the absorber bottom to the upper portion of a first distillation column (recovery column) for further product purification. The product recovered from the upper portion of the recovery column is then sent to a second distillation column for further purification and recovery of product acrylonitrile or methacrylonitrile. The bottom stream obtained from the recovery column is sent to a stripper distillation column to recover crude acetonitrile which is a valuable coproduct.

In the practice of the present invention an automatic pressure control valve is installed in the recovery column overhead line to enable the top pressure of the recovery column to be increased by about 0.7 to 34.5 kPa (0.1 to 5 psi) above the design top recovery column pressure. Typically, the recovery column top pressure is between 108 to less than 136 kPa (1 to less than 5 psig) normally between 129 to 132 kPa (4 to 4.5 psig). In the preferred practice of the present invention, the recovery column top pressure is operated at a constant pressure which may vary from 136 to 170 kPa (5 psig to about 10 psig) preferably 139 to 153 kPa (5.5 to 7.5 psig). The improvement of the present invention is applicable to separate recovery and stripper tower designs as well as stacked recovery/stripper tower designs for the recovery of acrylonitrile or methacrylonitrile provided that tower hydraulics is limited by jet flooding . The pressure control valve employed may be obtained from any control valve manufacturer. It may be automatically actuated or manually controlled.

Preferably, the ammoxidation reaction is performed in a fluid bed reactor although other types of reactors such as transport line reactors are envisioned. Fluid bed reactors, for the manufacture of acrylonitrile are well known in the prior art. For example, the reactor design set forth in U.S. Patent No. 3,230,246, is suitable.

Conditions for the ammoxidation reaction to occur are also well known in the prior art as evidenced by U.S. Patent Nos. 5,093,299; 4,863,891; 4,767,878 and 4,503,001. Typically, the ammoxidation process is performed by contacting propylene or isobutylene in the presence of ammonia and oxygen with a fluid bed catalyst at an elevated temperature to produce the acrylonitrile or methacrylonitrile. Any source of oxygen may be employed. For economic reasons, however, it is preferred to use air. The typical molar ratio of the oxygen to olefin in the feed should range from 0.5:1 to 4:1, preferably from 1:1 to 3:1. The molar ratio of ammonia to olefin in the feed in the reaction may vary from between 0.5:1 to 5:1. There is really no upper limit for the ammonia-olefin ratio, but there is generally no reason to exceed a ratio of 5:1 for economic reasons.

The reaction is carried out at a temperature of between the ranges of about 260° to 600°C, but the preferred ranges being 310° to 500°C, especially preferred being 350° to 480°C. The contact time, although not critical, is generally in the range of 0.1 to 50 seconds, with preference being to a contact time of 1 to 15 seconds.

In addition to the catalyst of U.S. Patent No. 3,642,930, other catalysts suitable for the practice of the present invention are set forth in U.S. Patent No. 5,093,299.

The conditions under which the absorber column, recovery column and stripper column are maintained range between 136 to 150 kPa (5 to 7 psig) (27 to 43°C) (80°F to 110°F), 108 to 132 kPA (1 to 4.5 psig) (68 to 77°C) (155°F to 170°F), and 150 to 191 kPa (7 to 13 psig) (77 to 99°C) (170°F to 210°F), respectively.

The improvement of the present invention is distinguished over the prior art by operating the recovery column by mechanical adjustment at a pressure which is above its normal designed pressure during the practice of the ammoxidation process for the manufacture of acrylonitrile/methacrylonitrile. Conventional procedures utilized a recovery column top pressure of less than 136 kPa (5 psig) In the preferred practice of the present invention, the recovery column top pressure is typically operated at a pressure ranging from 136 to 170 kPa (5 to 10 psig), preferably between 140 to 150 kPa (5.5 to 7.0 psig).

Operation of the recovery and purification process of the present invention results in the ability to achieve high throughput rates through the reactor without making any modifications to the recovery and purification system which involve capital expenditures. The present invention not only results in an unexpected improvement in the production rates but achieves this improvement without increasing the size of the towers utilized in the recovery and purification section. In addition, the attendant increase in production rates does not come with any observed deterioration in the performance of the absorber column during the recovery or the acrylonitrile or methacrylonitrile. The present invention results in improved efficiency of operation, improved throughput, increased productivity without the necessity of expenditure of capital.

## Claims

1. A process for the recovery of acrylonitrile or methacrylonitrile obtained from the reactor effluent of an ammoxidation reaction of propylene or isobutylene comprising passing the reactor effluent through an absorber column, a recovery column having a design top pressure in the range 108 to less than 136 kPa (1 to less than 5psig) and a stripper column **characterised in that** the hydraulic capacity of the recovery and stripper columns is improved by operating the recovery column at a top pressure which has been increased by mechanical means to between 0.7 to 34.5 kPa (0.1 to 5 psi) above its designed top pressure.

2. The process of claim 1 wherein the reactor effluent is obtained from the reaction of propylene, ammonia and an oxygen-containing gas in a fluid bed reactor which is in contact with a fluid bed ammoxidation catalyst.

3. The process of claim 1 wherein the reactor effluent is obtained from the reaction of isobutylene, ammonia and an oxygen-containing gas in a fluid bed reactor while in contact with a fluid bed ammoxidation catalyst.

4. The process of any one of the preceding claims wherein the recovery column is maintained at a top pressure of between 136 to 170 kPa (5 to 10psig).

5. The process of claim 4 wherein the recovery column top pressure is maintained between 139 to 150 kPa (5.5 to 7.0 psig).

6. The process of any one of claims 1 to 3 wherein the recovery column top pressure is increased by between 1.7 to 34.5 kPa (0.25 to 5 psi).

7. The process of claim 6 wherein the recovery column top pressure is increased by between 3.5 to 34.5 kPa (0.5 to 5psi).

8. The process of claim 7 wherein the recovery column top pressure is increased by between 7 to 34.5 kPa (1.0 to 5psi)

9. The process of claim 1 wherein the mechanical means comprises a pressure control means located on the overhead line of the recovery column.

10. The process of any one of claims 1 to 9 wherein the absorber column operates at a lean water to product ratio of 12:1 to 11:1.

## Patentansprüche

1. Verfahren zur Gewinnung von Acrylnitril oder Methacrylnitril, erhalten aus dem Reaktorabfluß einer Ammonoxidationsreaktion von Propylen oder Isobutylen, umfassend das Leiten des Reaktorsabflusses durch eine Absorbersäule, eine Gewinnungssäule mit einem Auslegungsoberdruck in dem Bereich von 108 bis weniger als 136 kPa (1 bis weniger als 5 psig) und eine Abtriebssäule, **dadurch gekennzeichnet, daß** die hydraulische Kapazität der Gewinnungs- und Abtriebssäule verbessert wird, indem die Gewinnungssäule bei einem Oberdruck betrieben wird, der durch mechanische Mittel auf 0,7 bis 34,5 kPa (0,1 bis 5 psi) über ihren Auslegungsoberdruck erhöht worden ist.

2. Verfahren nach Anspruch 1, wobei der Reaktorabfluß aus der Reaktion von Propylen, Ammoniak und einem Sauerstoff-enthaltenden Gas in einem Fließbettreaktor erhalten wird, der mit einem Fließbettammonoxidationskatalysator in Kontakt ist.

3. Verfahren nach Anspruch 1, wobei der Reaktorabfluß aus der Reaktion von Isobutylen, Ammoniak und einem Sauerstoff-enthaltenden Gas in einem Fließbettreaktor erhalten wird, während er mit einem Fießbettammonoxidationskatalysator in Kontakt ist.

4. Verfahren nach einem der vorhergehenden Ansprüche, wobei die Gewinnungssäule bei einem Oberdruck von 136 bis 170 kPa (5 bis 10 psig) gehalten wird.

5. Verfahren nach Anspruch 4, wobei der Gewinnungssäulen-Oberdruck zwischen 139 und 150 kPa (5,5 bis 7,0 psig) gehalten wird.

6. Verfahren nach einem der Ansprüche 1 bis 3, wobei der Gewinnungssäulen-Oberdruck um 1,7 bis 34,5 kPa (0,25 bis 5 psi) erhöht wird.

7. Verfahren nach Anspruch 6, wobei der Gewinnungssäulen-Oberdruck um 3,5 bis 34,5 kPa (0,5 bis 5 psi) erhöht wird.

8. Verfahren nach Anspruch 7, wobei der Gewinnungssäulen-Oberdruck um 7 bis 34,5 kPa (1,0 bis 5 psi) erhöht wird.

9. Verfahren nach Anspruch 1, wobei das mechanische Mittel ein Druckkontrollmittel umfaßt, das sich an der Oberleitung der Gewinnungssäule befindet.

10. Verfahren nach einem der Ansprüche 1 bis 9, wobei die Absorbersäule bei einem Verhältnis von armem Wasser zu Produkt von 12 : 1 bis 11 : 1 arbeitet.

## Revendications

1. Procédé de récupération d'acrylonitrile ou de méthacrylonitrile obtenu à partir de l'effluent de réacteur d'une réaction d'ammoxydation de propylène ou d'isobutylène, comprenant le passage de l'effluent de réacteur à travers une colonne d'absorption, une colonne de récupération ayant une pression en tête de conception dans la plage de 108 à moins de 136 kPa (de 1 à moins de 5 psig) et une colonne d'épuisement, **caractérisé en ce que** la capacité hydraulique des colonnes de récupération et d'épuisement est améliorée en faisant fonctionner la colonne de récupération à une pression de tête qui a été augmentée par des moyens mécaniques à une valeur de 0,7 à 34,5 kPa (0,1 à 5 psi) au-dessus de sa pression de tête de conception.

2. Procédé selon la revendication 1, dans lequel l'effluent de réacteur est obtenu à partir de la réaction de propylène, d'ammoniac et d'un gaz contenant de l'oxygène dans un réacteur à lit fluide qui est en contact avec un catalyseur d'ammoxydation à lit fluide.

3. Procédé selon la revendication 1, dans lequel l'effluent de réacteur est obtenu à partir de la réaction d'isobutylène, d'ammoniac et d'un gaz contenant de l'oxygène dans un réacteur à lit fluide tandis qu'il est en contact avec un catalyseur d'ammoxydation à lit fluide.

4. Procédé selon l'une quelconque des revendications précédentes, dans lequel la colonne de récupération est maintenue à une pression de tête allant de 136 à 170 kPa (5 à 10 psig).

5. Procédé selon la revendication 4, dans lequel la pression de tête de la colonne de récupération est maintenue à une valeur allant de 139 à 150 kPa (5,5 à 7,0 psig).

6. Procédé selon l'une quelconque des revendications 1 à 3, dans lequel la pression de tête de la colonne de récupération est augmentée de 1,7 à 34,5 kPa (0,25 à 5 psi).

7. Procédé selon la revendication 6, dans lequel la pression de tête de la colonne de récupération est augmentée de 3,5 à 34,5 kPa (0,5 à 5 psi).

8. Procédé selon la revendication 7, dans lequel la pression de tête de la colonne de récupération est augmentée de 7 à 34,5 kPa (1,0 à 5 psi).

9. Procédé selon la revendication 1, dans lequel les moyens mécaniques comprennent des moyens de régulation de la pression situés sur la ligne aérienne de la colonne de récupération.

10. Procédé selon l'une quelconque des revendications 1 à 9, dans lequel la colonne d'absorption fonctionne selon un rapport eau propre/produit de 12/1 à 11/1.
